# EUROPEAN PATENT APPLICATION

(11) **EP 2 572 731 A1**
(43) Date of publication of application: **27.03.2013**
(21) Application number: 11182792.9
(22) Date of filing: 26.09.2011
(51) Int. Cl.: A61K 47/02, A61K 47/12, A61K 47/34, A61K 9/14, A61K 9/16

(54) **Formulations based on solid dispersions**

(71) Applicant: Abbott GmbH & Co. KG, 65205 Wiesbaden (DE)
(72) Inventor: Kyeremateng, Samuel, 68163 Mannheim (DE); Woehrle, Gerd, 69214 Eppelheim (DE); Warnecke, Svenja, 23866 Nahe (DE); Kullmann, Simon, 67433 Neustadt (DE); Westedt, Ullrich, 69198 Schriesheim (DE); Weis, Jürgen, 60433 Frankfurt (DE)
(74) Representative: Reitstötter - Kinzebach

(57) **Abstract**

The present invention relates to formulations comprising a solid dispersion product of an active agent having at least one of a hydrogen bond donor moiety and a proton donor moiety and a pharmaceutically acceptable polyvinyllactam polyvinylacetate poly(alkylene glycol) graft copolymer, and to methods for preparing such formulations.

## Description

The present invention relates to formulations comprising a solid dispersion product of an active agent having at least one hydrogen bond donor moiety or proton donor moiety and a pharmaceutically acceptable polyvinyllactam polyvinylacetate poly(alkylene glycol) graft copolymer, and methods for preparing such formulations.

Pharmaceutical scientists increasingly face the challenge of delivering poorly water soluble drugs. To meet this challenge, attempts have been made to use amorphous solids in place of crystals in pharmaceutical formulations. Amorphous solids are preferred physical forms because they dissolve more rapidly than crystalline solids when contacted with a liquid medium such as gastric fluid. The ease of dissolution may be attributed at least in part to the fact that the energy required for dissolution of an amorphous drug is less than that required for the dissolution of a crystalline or microcrystalline solid phase.

Provisions must be made to stabilize amorphous drugs and counteract their tendency to undergo physical and chemical changes. One way of stabilizing the amorphous state of a drug involves forming solid solutions of the drug in polymeric matrices.

Water-soluble or water-dispersible polyvinyllactam polyvinylacetate poly(alkylene glycol) graft copolymers have been reported to be able to form solid dispersions with drugs, see WO 2007/051743 and WO 2009/013202. Such graft copolymers form flowable powders that can easily be mixed with liquid or solid active agents and processed by melt extrusion. In particular polyvinylcaprolactam polyvinylacetate poly(ethylene glycol) graft copolymer has shown excellent extrudability and easy processability. Polyvinyllactam polyvinylacetate poly(alkylene glycol) graft copolymers can provide transparent and clear solid dispersion products that are stable and resistant to recrystallization of the active agent dispersed therein. Moreover, in many cases such graft copolymers can improve solubility and bioavailability of poorly soluble drugs when used in solid dispersions.

Nevertheless, it has been observed by the present inventors that with certain active agents polyvinyllactam polyvinylacetate poly(alkylene glycol) graft copolymers form solid dispersions with only low dispersibility in aqueous media which impairs release and bioavailability of the dispersed active agent. This particularly applies to active agents having hydrogen bond donor moieties or proton donor moieties. It is believed that the polyvinyllactam polyvinylacetate poly(alkylene glycol) graft copolymer acts as a hydrogen bond acceptor which forms strong hydrogen bonds to the dispersed active agent.

A major aim of the present invention is to provide desired release profiles of active agents having hydrogen bond donor moieties or proton donor moieties from their solid dispersion products with polyvinyllactam polyvinylacetate poly(alkylene glycol) graft copolymers.

Surprisingly, it has now been found that this problem can be solved by incorporating an pH modifier into the solid dispersion product.

Thus, the invention provides a formulation comprising a solid dispersion product comprising formulation comprising a solid dispersion product comprising
(a) an active agent having at least one of a hydrogen bond donor moiety and a proton donor moiety,
(b) a pharmaceutically acceptable polyvinyllactam polyvinylacetate poly(alkylene glycol) graft copolymer, and
(c) a pharmaceutically acceptable pH modifier.

The invention further provides a method for producing a formulation as described herein, wherein an active agent having at least one hydrogen bond donor moiety or proton donor moiety, a pharmaceutically acceptable pH modifier and a pharmaceutically acceptable polyvinyllactam polyvinylacetate poly(alkylene glycol) graft copolymer are intimately blended.

The term "pharmaceutically acceptable", as used herein, refers to a compound that does not cause acute toxicity when the formulation of the invention comprising it is administered in an amount that is required for medical or cosmetic treatment or medical prophylaxis, or that is taken up by consumption of the maximum recommended intake of a nutritional product comprising the formulation of the invention. Expediently, all components of the formulation of the present invention are pharmaceutically acceptable.

As used herein, the term "pH modifier" refers to compounds capable of creating an alkaline ("alkaline pH modifier") or acidic ("acidic pH modifier") environment when dissolved in water.

The term "hydrogen bond donor moiety", as used herein, refers to a moiety that comprises a hydrogen atom attached to a relatively electronegative atom, such as an oxygen atom or a nitrogen atom, and does not dissociate (release a proton) in basic, aqueous solutions.

Exemplary hydrogen bond donor moieties are selected from primary amino, secondary amino, hydroxy, carbamoyl, thiocarbamoyl, sulfamoyl, sulfinamoyl and ureido.

The term "proton donor moiety", as used herein refers to a moiety that comprises an acidic hydrogen atom and is able to dissociate in a basic, aqueous solution releasing a proton. Preferably, the proton donor moieties of the active agents used in the present invention have pKa values from 3.0 to 5.5, for example from 4.0 to 5.0.

The proton donor moiety may be an organic acid moiety, wherein the acidic hydrogen is bound to a heteroatom such as oxygen or nitrogen. Examples of such proton donor moieties include carboxy, sulfo and sulfino; carboxy being particularly preferred.

Alternatively, the proton donor moiety may be a CH-acidic moiety. The acidic hydrogen atom in CH-acidic moieties is bound to a carbon atom adjacent, i.e. in alpha position, to a strongly electron withdrawing group, such as a carbonyl (e.g., in an ester, ketone or aldehyde), sulfonyl, cyano, trifluoromethyl or nitro group, which exerts an inductive effect that polarises the bond between the alpha-carbon atom and the thus acidic hydrogen atom. CH-acidic moieties include moieties, which, when deprotonized, form resonance-stabilized anions.

In preferred embodiments, the active agent has at least one carboxy group or CH-acidic moiety.

In one embodiment of the invention, the active agent is a non-ionic compound.

In particular, the active agent(s) comprised in solid dispersion product described herein may be selected from pharmaceutically active agents, cosmetically active agents and nutritional supplements.

The invention is particularly useful for water-insoluble or poorly water-soluble (or "lipophilic") compounds. Compounds are considered water-insoluble or poorly water-soluble when their solubility in water at 25°C (at pH 7.0) is 1 g/100 ml or less, in particular when it is 0.1 g/100 ml, 0.05 g/100 ml or even 0.01 g/100 ml, or less.

Examples of pharmaceutically active agents according to the invention include, but are not limited to:
(RS)-2-(4-(2-methylpropyl)phenyl)propanoic acid [ibuprofen],
2-{4-[(4-chlorophenyl)carbonyl]phenoxy}-2-methylpropanoic acid [fenofibric acid],
(2S)-2-(6-methoxynaphthalen-2-yl) propanoic acid [naproxen]
and stereochemically isomeric forms thereof.

The term "stereochemically isomeric forms" defines all possible stereoisomeric forms which the active ingredients may possess. In particular, stereogenic centers may have the R- or S-configuration and active ingredients containing one or more double bonds may have the E- or Z-configuration.

The solid dispersion product described herein may comprise from about 1 up to 60 wt%, for example up to 40 wt%, up to 30 wt%, up to 20 wt%, or from about 1 up to about 10 wt%, of active agent(s) relative to the total weight of the product.

The pharmaceutically acceptable polyvinyllactam polyvinylacetate poly(alkylene glycol) graft copolymer used in the solid dispersion product described herein is a thermoplastic polymer capable to act as a solid meltable solvent. It forms a matrix for dispersion, and in particular for dissolution, of the active agent(s) and the pH modifier(s). Preferably, said polymer is at least partly soluble or swellable in aqueous media, expediently under the conditions of use, in particular under physiological conditions in the digestive tract if the formulation is intended for oral administration. Most preferably, said polymer is a water-soluble polymer.

The term "graft copolymer" refers to a copolymer in which chains of a first polymer are grafted onto a second polymer chain. In other words, a graft copolymer has polymer chains of one kind "growing out" of the sides of polymer chains with a different chemical composition.

The polyvinyllactam polyvinylacetate poly(alkylene glycol) graft copolymer used in the solid dispersion product described is a copolymer, wherein chains of an N-vinyllactam/vinylacetate copolymer grow out of the sides of polyalkylene glycol chains.

Method of producing such graft copolymers are generally known in the art. They are obtainable by polymerization of N-vinyllactam and vinylacetate in the presence of an poly(alkylene glycol). Polymerization is preferably initiated by free radicals, and is preferably performed in solution in non-aqueous organic solvents or mixtures of non-aqueous and aqueous solvents. Suitable methods for producing polyvinyllactam polyvinylacetate poly(alkylene glycol) graft copolymer useful for the present invention are described, for example, in WO 2009/013202 and WO 2007/051743.

The pharmaceutically acceptable polyvinyllactam polyvinylacetate poly(alkylene glycol) graft copolymer used in the solid dispersion product described herein comprises
(i) 10 to 50 wt% poly(alkylene glycol) moieties,
(ii) 30 to 80 wt% N-vinyllactam moieties, and
(iii) 10 to 50 wt% vinyl acetate moieties.

Preferably, said graft copolymer comprises
(i) 10 to 35 wt% poly(alkylene glycol) moieties,
(ii) 30 to 70 wt% N-vinyllactam moieties, and
(iii) 15 to 35 wt% vinyl acetate moieties.

More preferably, said graft copolymer comprises
(i) 10 to 30 wt% poly(alkylene glycol) moieties,
(ii) 40 to 60 wt% N-vinyllactam moieties, and
(iii) 15 to 35 wt% vinyl acetate moieties.

It is particularly preferred that said graft copolymer comprises
(i) 10 to 20 wt% poly(alkylene glycol) moieties,
(ii) 50 to 60 wt% N-vinyllactam moieties, and
(iii) 25 to 35 wt% vinyl acetate moieties.

In each case the sum of (i), (ii) and (iii) makes up at least 95 wt%, at least 99 wt% and preferably 100 wt% of the total weight of the polyvinyllactam polyvinylacetate poly(alkylene glycol) graft copolymer.

The N-vinyllactam moieties of the graft copolymer may be N-vinylcaprolactam or N-vinylpyrrolidon moieties or mixtures thereof, and preferably are N-vinylcaprolactam moieties.

Poly(alkylene glycol) constitutes the backbone of the graft copolymer. Poly(alkylene glycols) having a number average molecular weight of from 1,000 to 100,000, from 1,500 to 35,000, or in particular from 1,500 to 10,000 are preferably used as grafting base. The molecular weights are determined based on the hydroxyl value determined according to DIN 53240. The alkyl moiety of the poly(alkylene glycol) may be selected from branched or linear C₁ to C₂₂ alkyl moieties, in particular C₁ to C₁₈ alkyl moieties such as methyl, ethyl, n-butyl, isobutyl, pentyl, hexyl, octyl, nonyl, decyl, dodecyl, tridecyl and octadecyl. For example, the poly(alkylene glycol) is selected from poly(ethylene glycols); poly(propylene glycols); polytetrahydrofurans; poly(butylene glycols) obtained from 2-ethyloxirane or 2,3-dimethyloxirane; copolymers obtained of ethylene oxide, propylene oxide and/or butylene oxides such as poly(ethylene glycol) poly(propylene glycol) block copolymers; or mixtures thereof. Preferably, the poly(alkylene glycol) is selected from poly(ethylene glycols) and mixtures thereof.

The graft copolymer used according to the invention suitably has a K value according to Fikentscher of from 10 to 60, preferably from 15 to 40 (determined in a 1 wt% solution in ethanol at 31-41°C).

In one embodiment of the invention, the polyvinyllactam polyvinylacetate poly(alkylene glycol) graft copolymer is a polyvinylcaprolactam polyvinylacetate poly(ethylene glycol) graft copolymer.

In a preferred embodiment of the invention, the polyvinyllactam polyvinylacetate poly(alkylene glycol) graft copolymer is a polyvinylcaprolactam polyvinylacetate poly(ethylene glycol) graft copolymer having a number average molecular weight determined by gel permeation chromatography in the range of 90,000 to 140,000 and a glass transitions temperature of 70°C such as Soluplus® (available from BASF AG, Ludwigshafen, Germany).

The solid dispersion product described herein comprises at least one pharmaceutically acceptable pH modifier; for example from 0.5 to 20 wt%, from 0.5 to 10 wt% or from 1 to 6 wt% pH modifier(s) per total weight of the solid dispersion product.

Preferably, the pH modifier used in the present invention is a water-soluble compound that is solid at ambient temperature.

According to one embodiment of the invention, the pharmaceutically acceptable pH modifier is an acidic pH modifier. Such acidic pH modifiers include pharmaceutically acceptable inorganic acids, e.g. sulfamic acid, and pharmaceutically acceptable organic acids, e.g. mono-, di- or polybasic carboxylic acids and mono-, di- or poly-sulfonic acids, as well as acidic salts thereof, e.g. acidic ammonium salts, acidic alkali metal salts and acidic alkaline earth metal salts of organic or inorganic acids.

Pharmaceutically acceptable carboxylic acids useful as pH modifiers for the present invention include aliphatic mono-, di- and tri-carboxylic acids, e.g. such having from 2 to 8 carbon atoms and in particular such having from 4 to 6 carbon atoms. Said carboxylic acids may be saturated or unsatured. Examples of suitable mono-carboxylic acids include sorbic acid, gluconic acid, lactic acid, glycolic acid and ascorbic acid. Examples of suitable di-carboxylic acids include adipic acid, malonic acid, succinic acid, glutaric acid, maleic acid, fumaric acid, malic acid, tartaric acid, tartronic acid, mucic acid, glutamic acid and aspartic acid. Examples of suitable tri-carboxylic acids include citric acid.

In particular embodiments of the invention, the acidic pH modifier is citric acid (C₆H₈O₇) or ascorbic acid (C₆H₈O₆).

According to another embodiment of the invention, the pharmaceutically acceptable pH modifier is an alkaline pH modifier. Examples of such alkaline pH modifiers include pharmaceutically acceptable basic salts of organic acids and inorganic acids, basic amino acids, metal oxides and metal hydroxides.

Suitable pharmaceutically acceptable basic salts of organic acids include basic alkali metal salts and basic alkaline earth metal salts of organic acids. Said organic acids may be the organic acids which are described herein as acidic pH modifiers.

Suitable pharmaceutically acceptable salts of inorganic acids include basic alkaline metal salts and alkaline earth metal salts of inorganic acids, and in particular basic salts of phosphoric acid or carbonic acid. For example, said salt may be selected from sodium carbonate, calcium carbonate, sodium hydrogen carbonate, potassium hydrogen carbonate, sodium hydrogen phosphate, and magnesium carbonate.

Suitable pharmaceutically acceptable metal oxides and metal hydroxides include basic alkaline metal oxides and alkaline earth metal oxides, or alkaline metal hydroxides and alkaline earth metal hydroxides, respectively. Examples of such compounds are magnesium oxide and magnesium hydroxide.

Suitable pharmaceutically acceptable basic amino acids include arginine and lysine, and basic salts thereof.

In particular embodiments of the invention the alkaline pH modifier is trisodium citrate (Na₃C₆H₅O₇), magnesium oxide (MgO) or sodium carbonate (Na₂CO₃).

The solid dispersion product described herein comprises a matrix of at least one graft copolymer (b), wherein at least one active agent (a) and at least one pH modifier (c) are homogeneously distributed. Preferably, the sum of components (a), (b) and (c) makes up at least 70 wt%, at least 80 wt%, at least 90 wt%, at least 95 wt%, at least 99 wt%, and most preferably 100 wt% of the solid dispersion product.

Various additives may be included in the formulation of the invention, for example lubricants, fillers, disintegrants, preservatives or stabilizers such as antioxidants, light stabilizers, radical scavengers and stabilizers against microbial attack, dyes such as azo dyes, organic or inorganic pigments such as iron oxides or titanium dioxide, or dyes of natural origin, as well as compounds which alter or mask flavor and/or odor of the formulation such as sweeteners, flavorings and odorants.

The matrix of the solid dispersion product is formed by the pharmaceutically acceptable polyvinyllactam polyvinylacetate poly(alkylene glycol) graft copolymer. It is particularly preferred that the active agent(s) in the solid dispersion product is/are present in an essentially non-crystalline state. This encompasses a state wherein essentially amorphous domains of active agent(s) are interspersed in the matrix, and a state wherein the active agent(s) are molecularly dispersed in the matrix. When said dispersion of the active agent(s) in the polymer phase is such that the system of active agent and polymer is chemically and physically uniform or homogeneous throughout, such a solid dispersion will be called a "solid solution" or a "molecular dispersion". The state of molecular dispersion corresponds to the maximum possible homogenization of the active agent in the polymer phase.

Known analytical methods can be used to investigate the state of such solid dispersions, for example differential scanning calorimetry (DSC) or wide angle X-ray scattering measurements (WAXS measurements). The DSC analytical measurement of an essentially non-crystalline state lacks the melting peak which occurs with the crystalline pure substance and is usually endothermic. Another possibility for identifying an essentially non-crystalline state is the reduction in intensity and/or absence of typical X-ray diffraction signals in the WAXS analysis.

A variety of methods for producing solid dispersions that can be applied for producing the solid dispersion product described herein are known in the art.

The solid dispersion product can be produced by blending at least one active agent having at least one hydrogen atom bound to an oxygen or a nitrogen atom, at least one pharmaceutically acceptable pharmaceutically acceptable pH modifier and at least one pharmaceutically acceptable polyvinyllactam polyvinylacetate poly(alkylene glycol) graft copolymer; heating the blend to obtain a homogeneous melt; and allowing the melt to solidify to obtain a solid dispersion product. The terms "melt" and "melting" should be interpreted broadly. For the purposes herein, these terms not only mean the alteration from a solid state to a liquid state, but can also refer to a transition to a glassy state or a rubbery state, and in which it is possible for one component of the mixture to get embedded more or less homogeneously into the other. In particular cases, one component will melt and the other component(s) will dissolve in the melt, thus forming a solution, which, upon cooling, may form a solid dispersion having advantageous dissolution properties. Blending and heating are conveniently performed in a mixer or kneader which is jacketed for heating.

A preferred method for producing the formulation of present invention comprises:
(a) blending the active agent(s); the pH modifier(s) and the graft copolymer(s);
(b) heating the blend to obtain a homogeneous melt;
(c) forcing the thus obtained melt through one or more nozzles; and
(d) allowing the melt to solidify to obtain a solid dispersion product.

Steps a) to c) may be performed in one or more than one apparatus suitable for this purpose, such as an extruder or kneader extruder. Preferably, the blend is subjected to a mixing action in a mixing section of the extruder.

Extruders are known per se. An extruder comprises a housing or barrel divided into several sections in a longitudinal direction. On the upstream side of the extruder, an opening is provided for feeding the active agent(s), the pH modifier(s) and the graft copolymer(s), and any further components such as the additives described herein. Usually, a hopper is placed on this opening so that the ingredients, usually in the form of powders, can be easily fed into the barrel of the extruder. The barrel ends in conveying direction in a die, where the dispersion is expelled.

The extruder comprises at least one rotating shaft. Alternatively, it may comprise two or up to six rotating shafts. The shafts may be co-rotating or counter-rotating. Processing elements disposed on adjacent shafts closely intermesh.

Each shaft carries a plurality of processing elements disposed axially one behind the other. The processing elements define a feeding and conveying section, at least one mixing section, and a discharging section. The feeding and conveying section is positioned farthest upstream, close to the hopper of the extruder, the at least one mixing section is positioned downstream of the feeding and conveying section, and the discharging section is positioned farthest downstream, close to the discharge opening of the extruder. The term "downstream" as used herein, refers to direction in which the material is being conveyed in the extruder, i.e. the conveying direction.

The processing elements of the feeding and conveying section as well as the discharging section are formed by screw-type elements. Preferably, these screw type elements form an endless screw having the feed direction and a uniform pitch flight. Thus, in the feeding and conveying section the powder is fed into the extruder and conveyed in the downstream direction.

In the mixing section(s) the material to be processed is homogenized by mixing or kneading. Paddle means or kneading blocks have conventionally been employed in kneading and plasticizing pharmaceutical mixtures. These kneading blocks consist of cam disks mutually offset at an angle in a peripheral direction. The cam disks have abutting faces that are perpendicular to the general conveying direction in the extruder. Alternatively, the mixing section(s) are defined by processing element(s) that comprise(s) a mixing element that is derived from a screw type element. A mixing element "being derived from a screw type element" is intended to mean an element whose basic shape is that of a screw element, but which has been modified such that it exerts a compounding or mixing effect in addition to a conveying effect. The underlying screw type element may have a positive-flight (positive-feed, "right-handed") screw element, may have a reverse-flight (negative-feed, "left-handed") screw element or a combination thereof. A preferred mixing element has a plurality of concentric ring portions formed by grooves turned into a screw type element. Therefore, the mixing element has a continuous screw flight, which is interrupted only by turned grooves with ring portions. Advantageously, the mixing element comprises screw portions between the ring portions which first cause a pressure buildup that forces the substance through the annular gap between the extruder housing and the ring portions with shearing action and elongation; the pressure is then reduced again.

The extruder shaft may further comprise one or more than one reverse-flight section(s), preferably arranged after the (last) mixing section and defined by reverse-flight elements. A reverse-flight element has a screw with a reverse-flight relative to the screw-type elements which may be arranged in the feeding and conveying section which define the general conveying direction of the extruder. Thus, the reverse-flight element convey the material in an opposite direction relative to the general conveying direction of the extruder and serves to create sufficient back-pressure to allow for a desired degree of mixing and/or homogenization. The reverse-flight element is designed to stow the material conveyed in the extruder. Therefore it may also be called a back-pressure element.

The substances which are fed to the extruder are melted in order to homogenize the melt and to disperse or, preferably, dissolve the active agent in the matrix efficiently. "Melting" means transition into a liquid or rubbery state in which it is possible for one component to be homogeneously embedded in the other. Melting usually involves heating above the softening point of the polymer. Usually, the maximum melt temperature is in the range of from 50 to 260°C, for example from 100 to 190°C, and is preferably not more to 160°C, e.g. not more than 140°C, or not more than 120°C. The maximum melt temperature that are optimal for forming the solid dispersion product depend on the composition of the mixture to be melt extruded, e.g. on the amount and melting point of the active agent to be incorporated into the product. At least part of the mixture must plasticizable at the temperature used. Expediently, a temperature should be chosen, where none of the components of the mixture to be melt extruded is decomposed.

The glass transition temperature and melt viscosity of the mixture to be melt extruded can be adjusted by adding thermoplastic polymers with a high glass transition temperature, for example polyvinylpyrrolidones, hydroxyalkylcelluloses or hydroxyalkylstarches. Plasticizers, for example propylene glycol or polyethylene glycol 400, may be added to achieve a lower glass transition temperature.

The extruder housing is heated in order to form a melt from the substances fed to the extruder. It will be appreciated that the working temperatures will also be determined by the kind of extruder or the kind of configuration within the extruder that is used. A part of the energy needed to melt, mix and dissolve the components in the extruder can be provided by heating elements, while the friction and shearing of the material in the extruder can also provide the mixture with a substantial amount of energy and aid in the formation of a homogeneous melt of the components.

In order to obtain a homogeneous distribution and a sufficient degree of dispersion of the active agent, the melt is kept in the heated barrel of the melt extruder for a sufficient length of time.

The extrudate exiting from the extruder ranges from pasty to viscous. Before allowing the extrudate to solidify, the extrudate may be directly shaped into virtually any desired shape. Shaping of the extrudate may be conveniently carried out by a calender with two counter-rotating rollers with mutually matching depressions on their surface. A broad range of tablet forms can be attained by using rollers with different forms of depressions. If the rollers do not have depressions on their surface, films can be obtained. Alternatively, the extrudate is moulded into the desired shape by injection-moulding. Alternatively, the extrudate is subjected to profile extrusion and cut into pieces, either before (hot-cut) or after solidification (cold-cut).

Optionally, the solid dispersion product resulting from such process of melt extrusion is milled or ground to granules. The granules may then be compacted. Compacting means a process whereby a powder mass comprising the granules is condensed under high pressure in order to obtain a compact with low porosity, e.g. a tablet. Compression of the powder mass is usually done in a tablet press, more specifically in a steel die between two moving punches.

Alternatively, the solid dispersion product can be produced by dissolving at least one active agent having at least one hydrogen atom bound to an oxygen or a nitrogen atom, at least one pharmaceutically acceptable pharmaceutically acceptable pH modifier and at least one pharmaceutically acceptable polyvinyllactam polyvinylacetate poly(alkylene glycol) graft copolymer in a suitable solvent, and then removing the solvent. A suitable solvent may be an organic solvent, for example ethanol, isopropanol, n-butanol, isobutanol, ethyl acetate, acetone and dimethylformamide. Any method of drying may be used for removing the solvent, for example spray drying, fluidized-bed drying, roller drying, supercritical drying, lyophilization, vacuum drying or evaporation. For oral administration of the formulations of the invention a variety of dosage forms may be used comprising granules, capsules, pellets, powders or tablets.

Granules consist of solid grains of formulations of the invention, each grain representing an agglomerate of powder particles. A lubricant is preferably used in compacting the granules. Suitable lubricants are selected from polyethylene glycol (e.g., having a Mw of from 1,000 to 6,000), magnesium and calcium stearates, sodium stearyl fumarate, and the like. The user can be offered single-dose preparations, for example granules packed in a small bag (sachet), a paper bag or a small bottle, or multidose preparations which require appropriate dimensions. However, in many cases, such granules do not represent the actual drug form, but are intermediates in the manufacture of particular drug forms, for example tablet granules to be compressed to tablets, capsule granules to be packed into hard gelatin capsules, or instant granules or granules for oral suspension to be put in water before intake.

As capsules, the formulations of the invention are usually packed into a hard shell composed of two pieces fitted together or a soft, one-piece, closed shell, which may vary in shape and size. It is likewise possible for formulations of the invention to be encased or enveloped or embedded in a matrix in suitable polymers, i.e. microcapsules and microspherules. Hard and soft capsules consist mainly of gelatin, while the latter have a suitable content of plasticizing substances such as glycerol or sorbitol. Hard gelatin capsules are used to receive formulations of the invention which have a solid consistency, for example granules, powder or pellets. Soft gelatin capsules are particularly suitable for formulations with a semisolid consistency and, if required, also viscous liquid consistency.

Pellets are granules of formulations of the invention in the particle size range from about 0.5 to 2 mm in diameter. Both with a narrow particle size distribution, preferably from 0.8 to 1.2 mm, and with an essentially round shape, are preferred.

Tablets are solid preparations in particular for oral use. The meaning of "oral administration" within the framework of the present invention is, in particular, that of the term "peroral administration" or "ingestion", thus the tablets are for absorption or action of the active agent in the gastrointestinal tract. Particular embodiments are coated tablets, layered tablets, laminated tablets, tablets with modified release of the active agent, matrix tablets, effervescent tablets, chewable tablets or pills. The formulations of the invention usually comprise at least a part of the necessary tablet excipients, such as binders, fillers, glidants and lubricants, and disintegrants. Tablets of formulations of the invention may also, if necessary, comprise other suitable excipients, for example excipients which assist tableting such as lubricants and glidants, e.g. talc and silicones, animal or vegetable fats, especially in hydrogenated form and those which are solid at room temperature. Coated tablets additionally comprise suitable coating materials, for example film coating agents with coating aids, especially those mentioned below. Coated tablets include, in particular, sugar-coated tablets and film-coated tablets.

Powders are finely dispersed solids of formulations of the invention with particle sizes usually of less than 1 mm. The above statements about granules apply correspondingly.

The solid dispersion products described herein have a higher dispersion rate in aqueous media and a higher release rate of the comprised active agent(s) into the aqueous media compared to solid dispersions which do not comprise pH modifier(s). Release of active agents from solid dispersion products may be determined according to chapter <711> Dissolution of United States Pharmacopeia (USP 33, 2010) using USP apparatus 2 (paddle) and 500 ml dissolution medium at a temperature of 37°C and a stirring speed of 50 rpm. Dissolved active agent(s) may be detected by means of H PLC and UVNis photometry.

The invention is further illustrated by the following, non-limiting, examples.

### EXAMPLE 1: Preparation of solid dispersion products using a DSC (differential scanning calorimetry) apparatus

The appropriate amounts of active agent (fenofibric acid or naproxen), pharmaceutically acceptable polyvinyllactam polyvinylacetate poly(alkylene glycol) graft copolymer (Soluplus®) and pharmaceutically acceptable pH modifier (trisodium citrate, sodium carbonate, magnesium oxide or citric acid) were weighed into a sample vessel to give a total weight of about 200 mg for each sample. After addition of approximately 0.50 to 0.75 ml tetrahydrofuran each sample was stirred for 2 h. A few drops of deionized water were added to samples containing sodium carbonate or trisodium citrate to facilitate dissolution of the pH modifier. The samples were then evaporated to dryness *in vacuo* at room temperature to form solid dispersion films. Approximately 70 mg of each solid dispersion film was collected, loaded into a 160 µl aluminum DSC pan and heated to 154°C at a heating rate of 3°C/min. Thus, cylindrical solid dispersion samples were obtained.

### EXAMPLE 2: Preparation of solid dispersion products by hot melt extrusion

The appropriate amounts of active agent (fenofibric acid or naproxen), pharmaceutically acceptable polyvinyllactam polyvinylacetate poly(alkylene glycol) graft copolymer (Soluplus®, available from BASF AG, Ludwigshafen, Germany) and pharmaceutically acceptable pH modifier (trisodium citrate, sodium carbonate, magnesium oxide or citric acid) for each sample and 1 wt% Aerosil were mixed. The mixtures were processed by hot melt extrusion using an extruder with the temperature profile Z1=110°C, Z2= 170°C, Z3=170°C.

### EXAMPLE 3: Dispersibility of solid dispersion products

Solid dispersion products were prepared from fenofibric acid and Soluplus® with or without the pH modifier trisodium citrate. The compositions of said solid dispersion products are indicated in Table 1. Dispersibility of the solid dispersion products was determined in de-ionized water at room temperature.

**Table 1: Compositions of solid dispersion products**

| | Reference product | Product #1 | Product #2 |
|---|---|---|---|
| fenofibric acid | 10 wt% | 10 wt% | 10 wt% |
| Soluplus® | 90 wt% | 88 wt% | 85 wt% |
| trisodium citrate | - | 2 wt% | 5 wt% |

While products #1 and #2 were completely dispersed after 4 h or 0.5 h, respectively, no dispersion of the reference product was observed even after 24 h.

### EXAMPLE 4: Drug release from solid dispersion products

Solid dispersion products were prepared from fenofibric acid ("Feno acid") or naproxen, and Soluplus® with or without a pH modifier selected from citric acid, sodium carbonate and magnesium oxide. Of each solid dispersion product a cylindrical sample of about 70 mg was placed in a vessel containing 75 ml de-ionized water or phosphate buffer (pH 6.8). The vessel was shaken on a Heidolph platform shaker at a rotation speed of 250 rpm and a temperature of 37°C. At regular time intervals a sample of 500 µl was taken from each solution, diluted with methanol and analyzed for the amount of diluted drug it contained by UV spectroscopy on a Shimadzu UV/Vis-1800 apparatus.

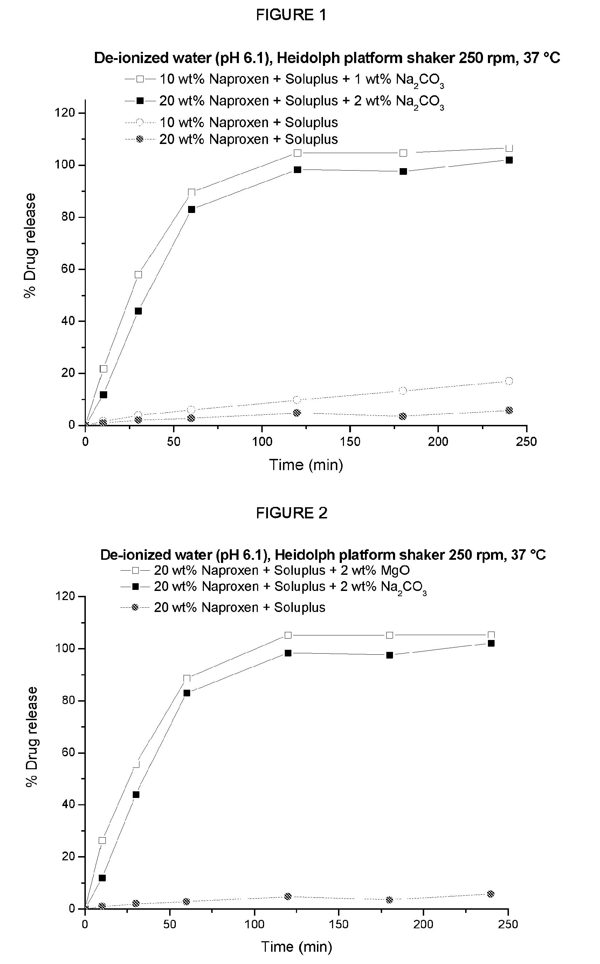

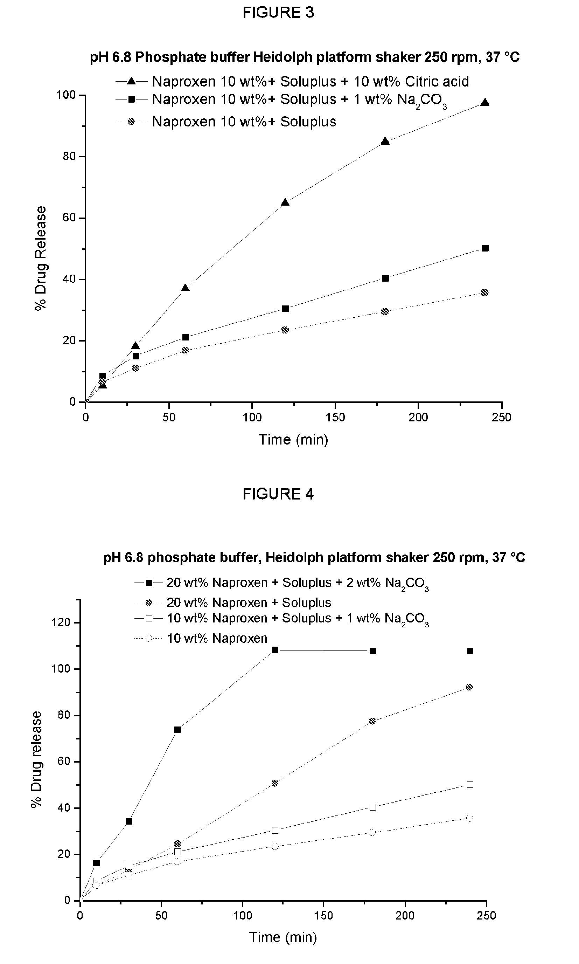

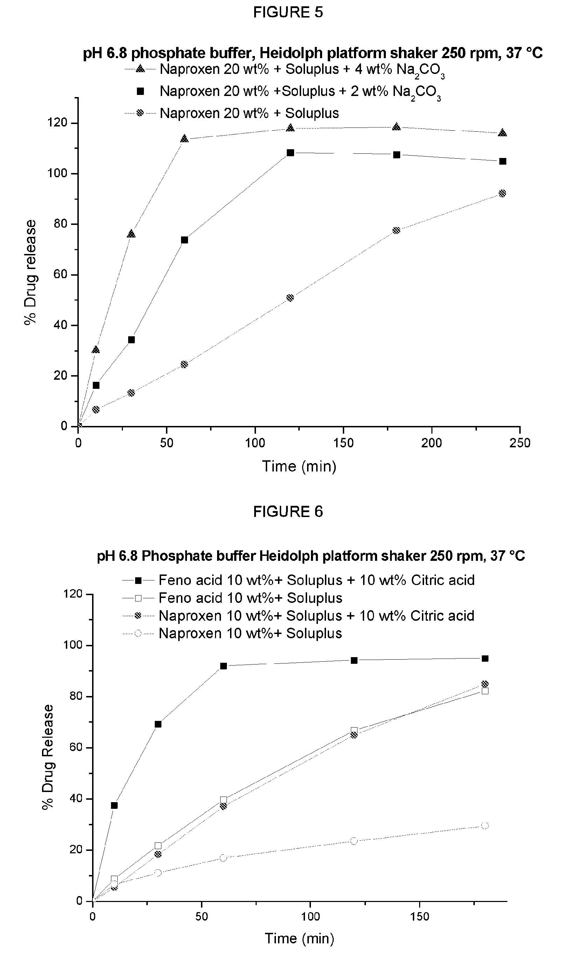

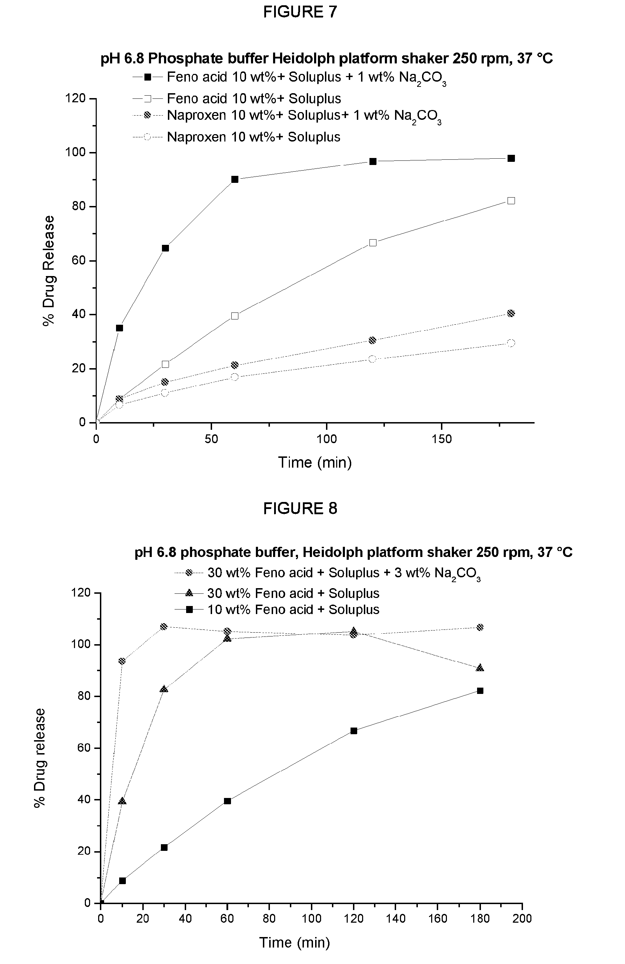

Each sample comprising a pH modifier showed a significantly improved rate of drug release compared to a corresponding sample without pH modifier (see Figures 1-8).

## Claims

1. A formulation comprising a solid dispersion product comprising
(a) an active agent having at least one of a hydrogen bond donor moiety and a proton donor moiety,
(b) a pharmaceutically acceptable polyvinyllactam polyvinylacetate poly(alkylene glycol) graft copolymer, and
(c) a pharmaceutically acceptable pH modifier.

2. The formulation of claim 1, wherein the active agent has at least one hydrogen bond donor moiety.

3. The formulation of claim 2, wherein the hydrogen bond donor moiety is selected from primary amino, secondary amino, hydroxy, carbamoyl, thiocarbamoyl, sulfamoyl, sulfinamoyl and ureido.

4. The formulation of claim 1, wherein the active agent has at least one proton donor moiety.

5. The formulation of claim 4, wherein the proton donor moiety is selected from organic acid moieties and CH-acidic moieties.

6. The formulation of any one of claims 1-5, wherein the active agent is non-ionic.

7. The formulation of any one of claims 1-6, wherein the active agent is a compound whose solubility in water at 25°C is 1 g/100 ml or less.

8. The formulation of claim 1, wherein the active agent is selected from (R*S*)-2-(4-(2-methylpropyl)phenyl)propanoic acid, 2-{4-[(4-chlorophenyl)carbonyl]phenoxy}-2-methylpropanoic acid and (2*S*)-2-(6-methoxynaphthalen-2-yl) propanoic acid.

9. The formulation of any one of claims 1-8, wherein the graft copolymer comprises
(i) 10 to 50 wt% poly(alkylene glycol) moieties,
(ii) 30 to 80 wt% N-vinyllactam moieties, and
(iii) 10 to 50 wt% vinyl acetate moieties.

10. The formulation of claim 9, wherein the N-vinyllactam moieties are N-vinylcaprolactam moieties.

11. The formulation of any one of claims 1-10, wherein the pH modifier is an alkaline pH modifier.

12. The formulation of claim 11, wherein the alkaline pH modifier is selected from basic salts of organic acids and inorganic acids, basic amino acids, metal oxides and metal hydroxides.

13. The formulation of any one of claims 1-10, wherein the pH modifier is an acidic pH modifier.

14. The formulation of claim 13, wherein the acidic pH modifier is selected from mono-, di- and polybasic carboxylic acids, mono-, di- and poly-sulfonic acids, and acidic salts thereof.

15. The formulation of any one of claims 1-14, comprising 0.5 to 20 wt% of the pH modifier relative to the weight of the solid dispersion product.

16. A method for producing the formulation of any one of claims 1-15, wherein the active agent having at least one hydrogen bond donor moiety or proton donor moiety, the pharmaceutically acceptable pH modifier and the pharmaceutically acceptable polyvinyllactam polyvinylacetate poly(alkylene glycol) graft copolymer are intimately blended.

17. The method of claim 16, comprising the steps of
(a) blending the active agent, the pH modifier and the graft copolymer;
(b) heating the blend to obtain a homogeneous melt;
(c) forcing the thus obtained melt through one or more nozzles; and
(d) allowing the melt to solidify to obtain a solid dispersion product.

18. The method of claim 17, wherein step (b) is carried out in an extruder and the blend is subjected to a mixing action in a mixing section of the extruder.
